# EUROPÄISCHE PATENTSCHRIFT

(11) **EP 0 490 325 B1**
(45) Veröffentlichungstag und Bekanntmachung des Hinweises auf die Patenterteilung: **06.09.1995**
(21) Anmeldenummer: 91121111.8
(22) Anmeldetag: 09.12.1991
(51) Int. Cl.: A61M 25/00

(54) **Ureterkatheter**
Urethral catheter
Cathéter urétral

(30) Priorität: 07.12.1990 EP 90123499
(43) Veröffentlichungstag der Anmeldung: 17.06.1992
(73) Patentinhaber: WILLY RÜSCH AG, D-71385 Kernen (DE)
(72) Erfinder: Ahmadzadeh, Massoud,Dr., DW-4445 Neuenkirchen (DE)
(74) Vertreter: KOHLER SCHMID + PARTNER

(56) Entgegenhaltungen:
- EP-A- 0 208 841
- EP-A- 0 230 040
- EP-A- 0 363 581
- EP-A- 0 370 785
- DE-C- 321 666
- FR-A- 1 211 941
- US-A- 4 593 687
- US-A- 4 784 639

## Beschreibung

Die Erfindung geht aus von einem Ureterkatheter mit einem länglichen Schaft und einem Lumen für die Drainage, dessen nierenbeckenseitiges Ende in einen einrollbaren Schaftabschnitt mit mehreren Durchtrittsöffnungen übergeht, die mit dem Lumen in Verbindung stehen, und einem blasenseitigen offenen Ende, das einen Ventilmechanismus zur Vermeidung des Refluxes von Körperflüssigkeit aus der Blase in die Niere aufweist, wobei der Ventilmechanismus aus einem trichterförmigen, dünnen und hochflexibel ausgebildeten Material besteht, welcher mit dem sich verjüngenden Teil der trichterförmigen Ausbildung am blasenseitigen offenen Ende befestigt ist, und das sich erweiternde freie Ende der trichterförmigen Ausbildung eine druckabhängig verschließbare Öffnung aufweist.

Ein derartiger Harnleiterkatheter ist durch die EP-A-208841 bekanntgeworden.

Bekannte Ureterkatheter weisen zwei einrollbare Schaftenden auf, die den Katheter lagefixieren und ein unkontrollierbares Wandern ins Nierenbecken bzw. in die Blase verhindern. Diese Katheter überbrücken den Harnleiter und gewährleisten einen sicheren Harnfluß vom Nierenbecken in die Blase. Steigt der Flüssigkeitsdruck in der Blase an, so kann es zu einem Harnrückfluß und damit zu einem Druckanstieg im Pyelon kommen.

Bei einer natürlichen Blasenkontraktion und gesunden funktionsfähigen Harnleitern zieht sich der Detrusor bei gleichzeitigem Verschluß der Harnleitermündungen durch den funktionell assoziierten Musculus triagonalis zusammen, und es kommt zur Harnentleerung. Ein Harnrückfluß ins Pyelon kann ausgeschlossen werden.

Den unerwünschten Vorgängen des Refluxes und der Druckerhöhung kann durch ein Ventil im blasenseitigen Ende dieser bekannten Katheter entgegengewirkt werden. Nach der EP-A-208841 ist ein derartiges Ventil als flächenhaftes Lippenventil ausgebildet. Zwei hauchdünne folienartige Blättchen werden an ihren Längsrändern miteinander verbunden. Das eine sich meist verjüngende Ende ist über das blasenseitige Ende des Katheters gestülpt und dort an der Schaftwandung befestigt. Das andere davon wegweisende Ende der eine mit Längsschweißnaht versehene Tüte oder einen Trichter bildenden Blättchen weist querverlaufende Ränder auf, die nicht miteinander verbunden sind. Zwischen den folienartigen Blättchen ist das blasenseitige Ende des Katheters angeordnet. Fließt nun Harn aus dem blasenseitigen Ende des Katheters in die Blase, so kann dieser begrenzt von den Innenseiten der Blättchen durch die eine Öffnung begrenzenden Querränder der Blättchen in die Blase abfließen. Das blasenseitige Ende des Katheters, wie auch die ein Lippenventil bildenden Blättchen befinden sich bei gelegtem Ureterkatheter in der Blase. Steigt nun der Flüssigkeitsdruck in der Blase an (Miktion), so werden die folienartigen Blättchen aneinander gepreßt und verschließen die blasenseitige Öffnung des Katheters. Mit dieser Maßnahme kann die antirefluxive Funktion des natürlichen Ostiums uretris künstlich aufrechterhalten werden.

Weiterhin ist aus EP-A-208 841 eine transuretral plazierbare Ureterschiene bekannt, die zur Vermeidung eines Refluxes die Positionierung des caudalen Stentendes im Ureter unmittelbar vor dem Ostium zur Blase vorsieht. Fremdkörperreize im Ureter und eine daraus resultierende Irritation der Peristaltik mit der möglichen Folge einer Ektasie des Hohlsystems sind nicht auszuschließen.

Sofern die bekannten Ureterkatheter ein gerades in die Blase hineinragendes Ende aufweisen, klagen Patienten während der Miktion häufig über Flankenschmerzen. Die Blasenschleimhaut ist verstärkten Irritationen ausgesetzt und sie ist auch durch das blasenseitige Ende dieser Ureterkatheter verletzbar. Ferner besteht die Gefahr, daß sich das freie Ende in den blasenseitigen Harnröhrenbereich schiebt.

Der Erfindung liegt daher die Aufgabe zugrunde, das blasenseitige Ende bekannter Ureterkatheter derart weiterzubilden, daß bei einer Miktion einerseits die Blasenschleimhaut nicht irritiert wird, sich das blasenseitige Ende der Ureterkatheter immer lagegerecht in der Blase befindet und andererseits die sichere Funktionsfähigkeit des bekannten Ventilmechanismusses gegeben ist.

Diese Aufgabe wird erfindungsgemäß dadurch gelöst, daß das blasenseitige offene Ende einen faltenbalgartigen oder mit einer spiralförmigen Kontur versehenen Schaftabschnitt aufweist, daß der Schaft innerhalb des Ventilmechanismus ein Rückhaltesystem aufweist und daß das Rückhaltesystem aus längs des Lumens durchgängig gespaltenen sich aufspreizenden Schafthälften gebildet ist, die mittels eines Mandrins auf den Schaftdurchmesser verstreckbar sind.

Unter einem hochflexiblen faltenbalgartigen Schaftabschnitt werden auch Abschnitte eines Schaftes verstanden, die eine spiralförmige Kontur aufweisen. Dabei kann die Spirale in Abhängigkeit von der axialen Länge Abschnitte unterschiedlicher Steigungen aufweisen und die Faltentiefe kann längs des faltenbalgartigen Schaftabschnitts unterschiedlich sein.

Der erfindungsgemäße Ureterkatheter hat damit den wesentlichen Vorteil, daß sich das in die Blase hineinragende Ende einer sich veränderten Blasenkontur immer bestmöglich anpassen kann, weil der Ureterkatheter über die Länge des faltenbalgartigen Schaftabschnitts hochflexible Eigenschaften aufweist. Ist der faltenbalgartige Schaftabschnitt einer auf ihn einwirkenden beliebig gerichteten Kraftkomponente ausgesetzt, so wird er bei kleinstem Druck in eine Richtung ausgelenkt, die eine möglicherweise daraus resultierende Gegenkraft ausschließt, das heißt, der faltenbalgartige Schaftabschnitt wird immer in den noch freien Raum in der Blase ausweichen. Damit können Irritationen der Blasenschleimhaut weitgehend vermieden und einer erosiven Trigonits vorgebeugt werden. Gleichzeitig wird auch die antirefluxive Funktion des natürlichen Ostiums bestmöglich künstlich simuliert, weil an den faltenbalgartig aufgebauten Schaftabschnitt ein Ventilmechanismus zur Verhinderung des Harnrückflusses ins Pyelon leicht und sicher anpaßbar ist.

Wird der gut biegsame Schaftabschnitt dadurch gebildet, daß der Schaft über einen Längenabschnitt von einem Monofil spiralförmig umwickelt ist, so ist dieser Abschnitt sicher armiert. Das Drainagelumen ist auch immer dann durchgängig, wenn der Schaft im Bereich des blasenseitigen Endes gebogen ist. Die Spirale kann längs des Schaftes unterschiedliche Steigungen aufweisen, d. h., die einzelnen Spiralwindungen sind unterschiedlich weit voneinander beabstandet. Mit dieser Maßnahme kann der Flexibilitätsgrad des Schaftabschnittes in Abhängigkeit von der Länge des Schaftes beeinflußt werden. Unterschiedliche Faltentiefen im Schaftabschnitt haben ähnliche Auswirkungen auf den Flexibilitätsgrad des faltenbagartigen Schaftabschnittes.

Da der Ventilmechanismus aus einem trichterförmigen dünnen und hochflexiblen ausgebildeten Material besteht, wobei der Ventilmechanismus mit dem sich verjüngenden Teil der trichterförmigen Ausbildung am blasenseitigen offenen Ende befestigt ist, und da das sich erweiternde freie Ende der trichterförmigen Ausbildung eine druckabhängig verschließbare Öffnung aufweist, kann diese Art von Ausgestaltung eines Verschlußmechanismusses einfach und sicher am faltenbalgartigen Schaftabschnitt angebracht werden. Die folienartigen Blättchen, das heißt der gesamte Ventilmechanismus, werden auch nicht von einem sich einrollenden Ende möglicherweise in der Funktion gestört, weil der faltenbalgartige Schaftabschnitt ohne Krafteinwirkung gerade und ausgestreckt in der Blase an der Schleimhaut anliegt oder frei in das Hohlorgan hineinragt.

Ist der sich verjüngende Teil der trichterförmigen Ausbildung als Schulter ausgebildet, die materialverstärkt ist, so übernimmt der Ventilmechanismus noch eine weitere Funktion, nämlich die der Lagefixierung. Durch eine Versteifung der Blättchen im Bereich ihrer Befestigung am faltenbalgartigen Schaftabschnitt ist ein Wandern des erfindungsgemäßen Ureterkatheters in den Harnleiter nicht möglich. Die Schultern verhindern, daß sich das blasenseitige offene Ende des Ureterkatheters in den Harnleiter zurückziehen kann.

In weiterer Ausgestaltung der Erfindung sind die verstärkten Schultern aus eingearbeiteten Metall- und/oder Kunststoffstreifen gebildet.

Dies hat den Vorteil, daß hierbei auf Materialien zuückgegriffen werden kann, die bei der Herstellung und Verwendung des erfindungsgemäßen Katheters an sich eingesetzt werden. So ist es denkbar, daß zur Verstärkung in den Ventilmechanismus Spiralfedern als schulterbildende Mittel eingesetzt werden.

Weist das blasenseitige offene Ende ein zu einem Schloßteil eines Pushers komplementäres Schloßteil auf, so kann der erfindungsgemäße Ureterkatheter, wie in der deutschen Offenlegungsschrift 38 31 652 A1 beschrieben, sicher mit einem Pusher verbunden und durch das Entfernen des Mandrins selbsttätig von ihm wieder entkoppelt werden. Das am blasenseitigen Ende des erfindungsgemäßen Katheters angebrachte Schloßteil ist von den den Ventilmechanismus bildenden Folienblättchen umgeben. Sie schützen das Schloßteil.

Über das Rückhaltesystem ist gewährleistet, daß der erfindungsgemäße Ureterkatheter nur so weit im Harnleiter wandern kann, bis das Rückhaltesystem am Ostium uretris anliegt. Der faltenbalgartige Schaftabschnitt liegt dann inerhalb des Ureters und das blasenseitige Ende des erfindungsgemäßen Ureterkatheters kann sich ohne nennenswerte Gegenkraft den sich verändernden Konturen der Blase anpassen.

Die sich aufspreizenden Schafthälften bilden Fixierflügel, die zum einem den Ventilmechanismus in seiner Funktion verbessern und damit einem transluminalen, vesikorenalen Reflux vorbeugen und zum anderen wird einem unbeabsichtigten Wandern des Ureterkatheters im Harnleiter ebenfalls sicher entgegengewirkt.

Die Erfindung ist in der Zeichnung dargestellt und wird anhand von Ausführungsbeispielen in der Zeichnung näher erläutert. Es zeigen:
- Fig. 1: den Urogenitaltrakt eines Patienten mit Harnleitern, Nieren und Blase sowie einem Ureterkatheter, wie er im rechten Harnleiter positioniert ist, mit Ventil und ohne erfindungsgemäßes Rückhaltesystem;
- Fig. 2: ein blasenseitiges Ende eines Ureterkatheters mit einem Pusher und einem Mandrin, der die gezeigten Schaftabschnitte im Ventil ohne ein erfindungsgemäßes Rückhaltesystem zusammenhält;
- Fig. 3: einen Abschnitt eines blasenseitigen offenen Endes eines Ureterkatheters mit abgebogenenem Schaft im Bereich des faltenbalgartig ausgebildeten Schaftabschnittes, ohne ein erfindungsgemäßes Rückhaltesystem;
- Fig. 4: eine mit Körperflüssigkeit gefüllte Blase und ein blasenseitiges Ende eines Ureterkatheters, wie er aus einem Ostium uretris herausragt, ohne ein erfindungsgemäßes Rückhaltesystem;
- Fig. 5: eine zusammengezogene Blase mit einem aus einem Ostium uretris herausragenden Ende eines Ureterkatheters, mit Ventil und ohne ein erfindungsgemäßes Rückhaltesystem;
- Fig. 6: ein weiteres Ausführungsbeispiel einer zusammengezogenen Blase mit einem blasenseitigen Ende eines Ureters, wie er sich der Schleimhautkontur der Blase anpaßt, mit Ventil und ohne ein erfindungsgemäßes Rückhaltesystem;
- Fig. 7: einen erfindungsgemäßen Ureterkatheter mit einem innerhalb des Ventilmechanismus angeordneten Rückhaltesystem.
- Fig. 7a: eine vergrößerte Darstellung des Rückhaltesystems der Fig. 7.

Die einzelnen Figuren der Zeichnung zeigen den erfindungsgemäßen Gegenstand teilweise stark schematisiert und sind nicht maßstäblich zu verstehen. Die Gegenstände der einzelnen Figuren sind teilweise stark vergrößert dargestellt, damit ihr Aufbau besser gezeigt werden kann.

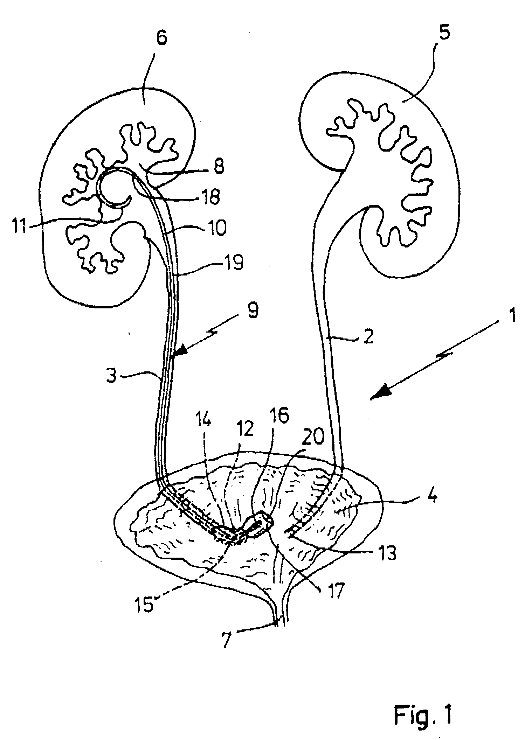

Fig. 1 zeigt mit 1 einen Urogenitaltrakt im Schnitt mit einem linken Harnleiter 2, einem rechten Harnleiter 3, einer Blase 4, einer linken Niere 5 und einer rechten Niere 6. Mit 7 ist der Verlauf der Harnröhre 7 aus der Blase angedeutet. Im Nierenbecken 8 der rechten Niere 6 ist ein Ureterkatheter 9 positioniert, der mit einem Schaft 10 im rechten Harnleiter 3 verläuft und sich über einen einrollbaren Schaftabschnitt 11 im Nierenbecken 8 in Position hält. Ein blasenseitiges Ende 12 des Ureterkatheters 9 ragt in die Blase 4. In der Figur ist das linke Ostium uretris mit 13 und das rechte Ostium uretris mit 14 gekennzeichnet. Durch das Ostium uretris 14 tritt das blasenseitige Ende 12 des Ureterkatheters 9 in die Blase 4. Dabei ragt ein faltenbalgartiger Schaftabschnitt 15 in die Blase 4 hinein, der je nach Blasenkontur an der Blasenschleimhaut anliegen kann. Der faltenbalgartige Schaftabschnitt 15 geht über in ein Schloßteil 16, das mit einem weiteren Schloßteil eines Pushers eine lösbare Verbindung eingehen kann. Das Schloßteil 16 ist in seinem Aufbau und Funktion in der deutschen Offenlegungsschrift DE 38 31 652 A1 beschrieben. Das Schloßteil 16 ist derart ausgestaltet, daß es die Öffnung des blasenseitigen Endes des Ureterkatheters 9 nicht beeinträchtigt. Das blasenseitige offene Ende 12 ist weiterhin mit einem Ventilmechanismus 17 versehen, der tütenartig das Schloßteil 16 ummantelt und zum freien Ende hin eine Öffnung aufweist. Im Bereich des faltenbalgartigen Schaftabschnittes 15 ist der Ventilmechanismus 17 mit dem Ureterkatheter 9 fest und flüssigkeitsundurchlässig verbunden.

Der Ureterkatheter 9 ist im Bereich des einrollbaren Schaftabschnittes 11 mit Durchtrittsöffnungen 18 versehen, die mit einem zentralen Lumen 19 in Verbindung stehen. Das zentrale Lumen 19 ist über die gesamte Länge des Schaftes 10 ausgebildet und steht in Verbindung mit einer Öffnung 20 des Ventilmechanismus 17.

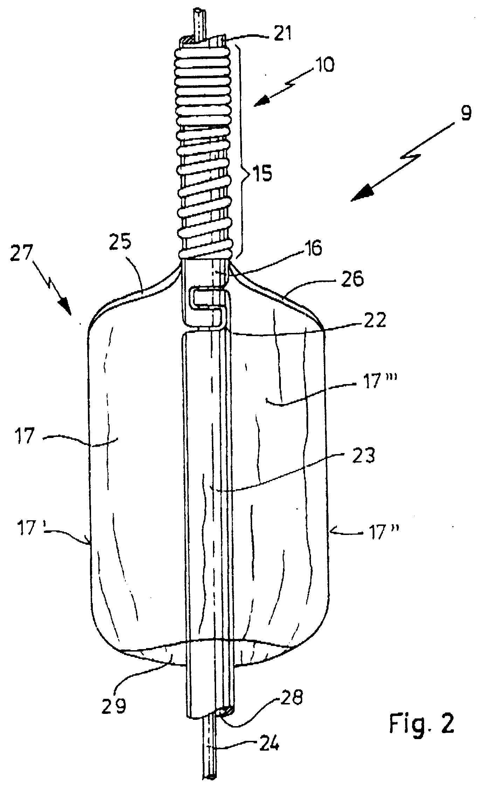

Fig. 2 zeigt mit dem blasenseitigen Ende 12 einen Längenabschnitt des Ureterkatheters 9. Ein Schaftabschnitt 21 ist Bestandteil des Schaftes 10. Der Schaft 10 geht zum blasenseitigen Ende hin über in den faltenbalgartigen Abschnitt 15, an den sich das Schloßteil 16 formschlüssig anschließt.

Der faltenbalgartige Abschnitt 15 weist an seiner äußeren Oberfläche abgerundete Falten auf, die sich über einen gewissen Längenabschnitt höckerförmig aneinanderreihen. Der Abschnitt 15 ist vom Schaftabschnitt 21 gebildet, der von einem Monofil spiralförmig umwickelt ist. Die vom Monofil gebildete Spirale weißt längs des Schaftabschnittes 21 eine unterschiedliche Steigung auf. In der Nähe des Ventilmechanismus 17 sind die einzelnen Spiralwindungen weit beabstandet, d. h., dieser Teil des Schaftabschnittes 21 ist in jedwede Richtung hochbiegsam. Durch das spiralförmig um den Schaftabschnitt 21 gewickelte Monofil ist der Schaft 10 in diesem Abschnitt armiert, d. h., das Drainagevolumen bleibt auch bei starken Biegungen des Schaftes 10 durchgängig. Zusätzlich kann die Faltentiefe im Abschnitt 15 mit unterschiedlich starken Monofilen beeinflußt werden, d. h., je tiefer und ausgeprägter die Falten desto flexibler ist der Schaft 10.

Die Ausbildung der Spirale in der Figur 2 ist beispielhaft zu verstehen. Weiterhin kann das Monofil mit dem Schaftmaterial einen Materialverbund eingehen.

Der Ventilmechanismus 17 ist hier von einer dünnen folienartigen Kunststoffschicht gebildet, die von der Schaftachse beabstandete und dazu längsverlaufende Längsfalze 17', 17'' aufweist und damit die Umhüllung in einen vorderen Flächenabschnitt 17''' und in einen hinteren Flächenabschnitt aufteilt. In der Fig. 2 greift ein komplementäres Schloßteil 22 in das Schloßteil 16. Das Schloßteil 22 ist Teil eines Pushers 23, der einen Mandrin 24 aufnehmen kann.

Der Ventilmechanismus 17 ist im Bereich zum faltenbalgartigen Schaftabschnitt 15 hin verstärkt, derart, daß die Verjüngung Schultern 25, 26 bildet. Die Schultern 25, 26 verstärken, versteifen in diesem Bereich das Folienmaterial des Ventilmechanismus 17. Mit den Schultern 25, 26 bildet der Ventilmechanismus 17 ein Rückhaltesystem 27, das verhindert, daß das blasenseitige Ende des Schaftes 10 im Harnleiter zur Niere hin wandern kann.

Sowohl der Pusher 23 wie auch der Schaft 10 mit dem Schaftabschnitt 21 weisen ein Lumen 28 auf, durch das der Mandrin 24 führbar ist. Der Mandrin 24 hält die Schloßteile 16 und 22 zusammen. Wird der Mandrin 24 zurückgezogen und werden die Schloßteile 16, 22 freigelegt, so öffnet sich die Verbindung selbsttätig und der Pusher 23 kann aus dem Bereich des Ventilmechanismus herausgezogen werden. Durch das Lumen 28 wird die Körperflüssigkeit aus dem Nierenbecken in die Blase geleitet.

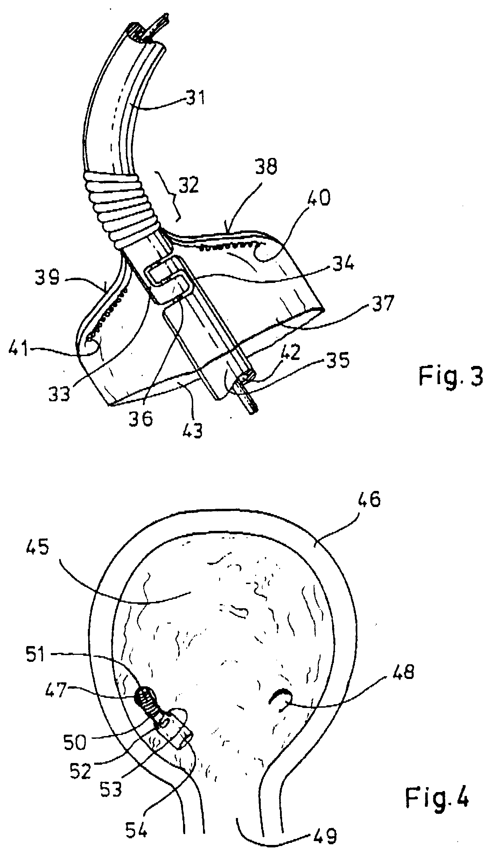

Fig. 3 zeigt eine weitere Ansicht eines Abschnittes eines blasenseitigen Endes eines erfindungsgemäßen Ureterkatheters. Ein Schaftabschnitt 31 ist gebogen gezeigt, und ein faltenbalgartiger Schaftabschnitt 32 ist auf einer Seite gestaucht und auf der anderen Seite geweitet. Man sieht deutlich in der Fig. 3, daß aufgrund der Falten eine Auslenkung des Schaftes 31 erleichtert wird. Ein Schloßteil 33 des Schaftabschnittes 31 greift in ein komplementäres Schloßteil 34 eines Pushers 35. Die Schloßteile 33, 34 werden von einem Mandrin 36 zusammengehalten. Die Schloßteile 33, 34 sind von einem Ventilmechanismus 37 umgeben, der ähnlich, wie schon beschrieben, aufgebaut ist. Die als Schultern 38, 39 ausgebildeten Teilbereiche des Ventilmechanismus 37 enthalten eine erste Spiralfeder 40 und eine zweite Spiralfeder 41, die zur Versteifung dieser Teilbereiche beitragen. Der in der Figur gezeigte Abschnitt des Mandrins 36 ist im Lumen 42 des Pushers 35 und des Schaftabschnittes 31 geführt.

Der Ventilmechanismus 37 ist an seinem freien Ende mit einer Öffnung 43 versehen, die sich flüssigkeitsdruckabhängig verschließen bzw. öffnen kann. Strömt Harn über den Schaftabschnitt 31 in den Ventilmechanismus 37, so kann der Harn über die Öffnung 43 in die Blase fließen.

Fig. 4 zeigt stark vereinfacht einen Querschnitt durch eine Blase 45 mit einem Musculus detrusor 46 und einem Ostium uretris 47 und 48. Bei einer Blasenkontraktion kann über eine Harnröhre 49 die Körperflüssigkeit abfließen. Aus dem Ostium uretris 47 ragt ein blasenseitiges Ende 50 eines Ureterkatheters, von dem ein faltenbalgartiger Schaftabschnitt 51 zu sehen ist. Schultern 52, 53 des Ventilmechanismus begrenzen die Verschiebbarkeit des Ureterkatheters in Richtung Nierenbecken. Über eine Öffnung 54 kann Harn aus dem Nierenbecken in die Blase 45 fließen.

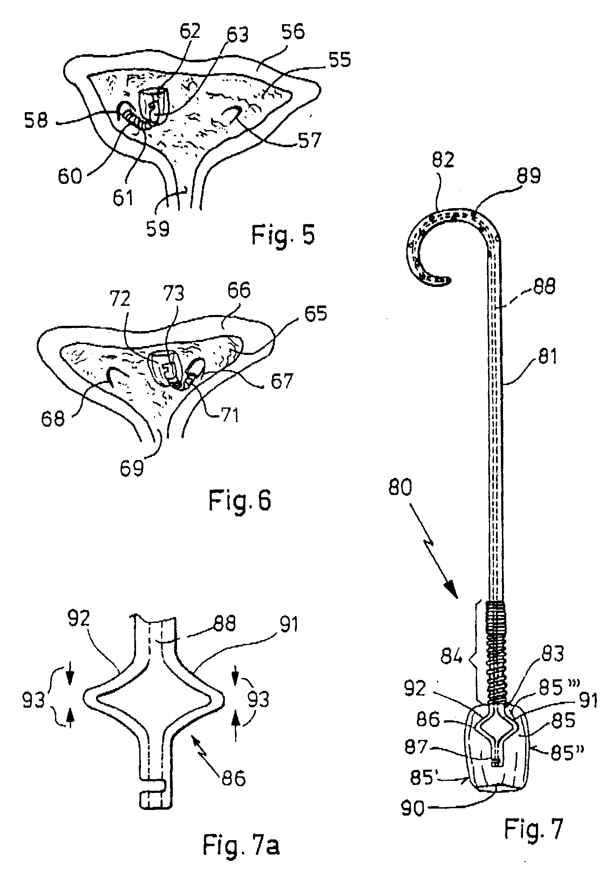

Fig. 5 zeigt eine weitgehend entleerte Blase 55 im Querschnitt mit einem Ostium uretris 57 und 58. Aus dem Ostium uretris 58 ragt ein blasenseitiges Ende des Ureterkatheters 60 in den noch verbleibenden Freiraum der Blase 55. Ein Ventilmechanismus 62 umgibt ein Schloßteil 53, das sich an den faltenbalgartigen Schaftabschnitt 61 anschließt und wie die schon angesprochenen Schloßteile eine Öffnung aufweist, die im Durchmesser weitgehend dem Durchmesser des Lumens des Ureterkatheters entspricht.

Fig. 6 zeigt in einer weiteren Ausführungsform eine im Querschnitt zusammengezogene Blase 65 mit einem Musculus detrusor 66 und einem Ostium uretris 67 und 68. Mit 69 ist die Harnröhre angedeutet. Ein faltenbalgartiger Schaftabschnitt 71 liegt an der Schleimhaut der Blase 65 an und paßt sich deren verändernden Konturen an. Ein Ventilmechanismus 72 ummantelt das Schloßteil 73.

Fig. 7 zeigt einen Ureterkatheter 80 mit einem Schaft 81, der in einen einrollbaren Schaftabschnitt 82 übergeht. Am blasenseitigen offenen Ende 83 ist ein faltenbalgartiger Schaftabschnitt 84 ausgebildet, an den sich der Ventilmechanismus 85 anschließt. Innerhalb des Ventilmechanismus 85 ist in den Schaft 81 ein Rückhaltesystem 86 eingearbeitet, das von Schafthälften 91, 92 gebildet ist. Das Rückhaltesystem 86 ist mittels eines Mandrins auf den Schaftdurchmesser verstreckbar. Wird der Mandrin zurückgezogen, so zieht sich der Schaft 81 im Bereich des Rückhaltesystems 86 zusammen und bildet an dieser Stelle eine Verdickung (Memory effect).

Der Ventilmechanismus 85 ummantelt zusätzlich ein Schloßteil 87.

Der Ventilmechanismus 85 weist eine Haubenform auf und ist an den Längsrändern mit Längsverschweißungen 85', 85'' versehen, damit sich der vordere Flächenabschnitt 85''' der Haube bei einem erhöhten Flüssigkeitsdruck in der Blase an den verdeckten hinteren Flächenabschnitt flächenhaft pressen kann. Von dem Schloßteil 87 ausgehend ist ein Lumen 88 im Schaft 81 des Ureterkatheters 80 bis zum nierenseitigen Schaftende, das verschlossen ist, ausgebildet. Durchtrittsöffnungen 89 stehen mit dem Lumen 88 in Verbindung. Tritt durch die Durchtrittsöffnungen 89 Körperflüssigkeit in das Lumen 88 ein, so kann es durch den Ventilmechanismus 85 hindurch über eine Öffnung 90 aus dem Ureterkatheter 80 in die Blase strömen.

Fig. 7a zeigt das Rückhaltesystem 86 in vergrößerter Darstellung. Auf die Darstellung des Ventilmechanismus 85 wurde verzichtet. Die Schafthälften 91, 92 werden dadurch gebildet, in dem man den Schaft 81 über einen Längenabschnitt im Bereich des Lumens 88 durchtrennt. Die Schafthälften 91, 92 ziehen sich in Pfeilrichtungen 93 eleastisch zusammen (Memory effect). Im zusammengezogenen Zustand bilden sich flächenhafte Fixierflügel, die mittels eines Mandrins entgegen den Pfeilrichtungen 93 auf den Schaftdurchmesser reduziert verstreckbar sind.

## Patentansprüche

1. Ureterkatheter mit einem länglichen Schaft (10; 81) und einem Lumen (19; 28; 42; 88) für die Drainage, dessen nierenbeckenseitiges Ende in einen einrollbaren Schaftabschnitt (11; 82) mit mehreren Durchtrittsöffnungen 18; 89) übergeht, die mit dem Lumen (19; 28; 42; 88) in Verbindung stehen, und einem blasenseitigen offenen Ende (12; 50; 60; 83), das einen Ventilmechanismus (17; 37; 62; 72; 85) zur Vermeidung des Refluxes von Körperflüssigkeit aus der Blase in die Niere aufweist, wobei der Ventilmechanismus (17; 37; 62; 72; 85) aus einem trichterförmigen, dünnen und hochflexibel ausgebildeten Material besteht, welcher mit dem sich verjüngenden Teil der trichterförmigen Ausbildung am blasenseitigen offenen Ende (12; 50; 60; 83) befestigt ist, und das sich erweiternde freie Ende der trichterförmigen Ausbildung eine druckabhängig verschließbare Öffnung (20; 29; 43; 54; 90) aufweist, dadurch gekennzeichnet,
daß das blasenseitige offene Ende (12; 50; 60; 83) einen faltenbalgartigen oder mit einer spiralförmigen Kontur versehenen Schaftabschnitt (15; 32; 51; 61; 71; 84) aufweist, daß der Schaft (10; 81) innerhalb des Ventilmechanismus (17; 37; 62; 72; 85) ein Rückhaltesystem (86) aufweist, und daß das Rückhaltesystem (86) aus längs des Lumens (88) durchgängig gespaltenen sich aufspreizenden Schafthälften (91, 92) gebildet ist, die mittels eines Mandrins auf den Schaftdurchmesser verstreckbar sind.

2. Ureterkatheter nach Anspruch 1, dadurch gekennzeichnet, daß der faltenbalgartige oder mit einer spiralförmigen Kontur versehene Schaftabschnitt (15; 32; 51; 61; 71; 84) aus einem den Schaft (10; 81) spiralförmig umwickelnden Monofil gebildet ist.

3. Ureterkatheter nach Anspruch 2, dadurch gekennzeichnet, daß die von dem Monofil gebildete Spirale längs des Schaftes (10; 81) unterschiedliche Steigungen aufweist.

4. Ureterkatheter nach einem der vorangehenden Ansprüche, dadurch gekennzeichnet, daß der sich verjüngende Teil der trichterförmigen Ausbildung Schultern (25, 26; 38, 39; 52, 53) bildet, die materialverstärkt sind.

5. Ureterkatheter nach Anspruch 4, dadurch gekennzeichnet, daß die verstärkten Schultern (25, 26; 38, 39; 52, 53) aus eingearbeiteten Metall- und/oder Kunststoffstreifen gebildet sind.

6. Ureterkatheter nach einem der vorangehenden Ansprüche, dadurch gekennzeichnet, daß das blasenseitige offene Ende (12, 50; 60; 83) ein zu einem Schloßteil (22; 34) eines Pushers (23, 35) komplementäres Schloßteil (16; 33; 63; 73; 87) aufweist.

## Claims

1. Ureteral catheter having an elongated shaft (10; 81) and a lumen (19; 28; 42; 88) for drainage, said shaft (10; 81) having a pigtail (11; 82) with several openings (18; 89) therein on its renal pelvis end, whereby said openings (18; 89) communicate with said lumen (19; 28; 42; 88) and said shaft (10; 81) has an open tip (12; 50; 60; 83) with a valve mechanism (17; 37; 62; 72; 85) on its bladder end for preventing return of body fluid from the bladder into the kidney, said valve mechanism (17; 37; 62; 72; 85) being made of a thin and highly flexible funnel-shaped material and being attached with its tapered part to said open tip (12; 50; 60; 83) of the bladder end and the enlarged free end of the tapered part comprises a pressure-dependent locking opening (20; 29; 43; 54; 90), characterized in that said open tip (12; 50; 60; 83) has a bellow-type or spiral-shaped shaft section (15; 32; 51; 61; 71; 84) on its bladder end and the shaft (10; 81) comprises a retainer system (86) within said valve mechanism (17; 37; 62; 72; 85), the retainer system (86) being formed by split half-shafts (91, 92) which are slotted along said lumen (88) and which can be stretched to the shaft diameter using a mandrin.

2. Ureteral catheter according to claim 1, characterized in that said bellow-type of spiral shaped shaft section (15; 32; 51; 61; 71; 84) is made of a monofil spirally wound about said shaft (10; 81).

3. Ureteral catheter according to claim 2, characterized in that the spiral formed by the monofil has different pitches along said shaft (10; 81).

4. Ureteral catheter according to one of the preceding claims, characterized in that the tapered section of the funnel-shaped part forms shoulders (25, 26; 38, 39; 52; 53) which are reinforced with material.

5. Ureteral catheter according to claim 4, characterized in that strips made out of metal and/or synthetic material are incorporated into the reinforced shoulders (25, 26; 38, 39; 52; 53).

6. Ureteral catheter according to one of the preceding claims, characterized in that the open tip (12; 50; 60; 83) of the bladder end comprises a first locking part (16; 33; 63; 73; 87) which is complementary to a second locking part (22; 34) of a pusher (23; 25).

## Revendications

1. Cathéter urétéral comportant une tige (10; 81) de forme allongée et une lumière (19; 28; 42; 88) pour le drainage, dont l'extrémité côté bassinet se raccorde à un tronçon de tige enroulable (11; 82) présentant plusieurs orifices de passage (18; 89) qui communiquent avec la lumière (19; 28; 42; 88), ainsi que, côté vessie, une extrémité ouverte (12; 50; 60; 83) pourvue d'un mécanisme de clapet (17; 37; 62; 72; 85) pour éviter le reflux de liquide corporel de la vessie dans le rein, le mécanisme de clapet (17; 37; 62; 72; 85) étant constitué d'un matériau en forme d'entonnoir, mince et très souple, qui est fixé par la partie qui se rétrécit de la conformation en entonnoir à l'extrémité ouverte côté vessie (12; 50; 60; 83), et l'extrémité libre évasée de la conformation en entonnoir présentant une ouverture (20; 29; 43; 54; 90) pouvant être fermée en fonction de la pression,
caractérisé en ce que
l'extrémité ouverte côté vessie (12; 50; 60; 83) présente un tronçon de tige (15; 32; 51; 61; 71; 84) semblable à un soufflet ou pourvu d'un concour spiralé, que la tige (10; 81) présente un système de retenue (86) à l'intérieur du mécanisme de clapet (17; 37; 62; 72; 85), et que le système de retenue (86) est formé de moitiés de tige (91, 92) fendues de façon continue le long de la lumière (88) et s'écartant l'une de l'autre, qui sont extensibles au moyen d'un mandrin, ce qui les ramène au diamètre de la tige.

2. Cathéter urétéral selon la revendication 1, caractérisé en ce que le tronçon de tige (15; 32; 51; 61; 71; 84) semblable à un soufflet ou pourvu d'un contour spiralé, est formé à l'aide d'un monofilament enroulé en spirale autour de la tige (10; 81).

3. Cathéter urétéral selon la revendication 2, caractérisé en ce que la spirale formée par le monofilament présente des pas différents dans le sens de la longueur de la tige (10; 81).

4. Cathéter urétéral selon une des revendications précédentes, caractérisé en ce que la partie qui se rétrécit de la conformation en entonnoir, forme des épaulements (25, 26; 38, 39; 52, 53) renforcés par un matériau de renfort.

5. Cathéter urétéral selon la revendication 4, caractérisé en ce que les épaulements renforcés (25, 26; 38, 39; 52, 53) sont formés de bandes de métal et/ou plastique incorporées.

6. Cathéter urétéral selon une des revendications précédentes, caractérisé en ce que l'extrémité ouverte côté vessie (12, 50; 60; 83) présente une partie d'accouplement (16; 33; 63; 73; 87) complémentaire à une partie d'accouplement (22; 34) d'un poussoir (23, 35).
